# EUROPEAN PATENT SPECIFICATION

(11) **EP 3 260 081 B1**
(45) Date of publication and mention of the grant of the patent: **22.05.2024**
(21) Application number: 16205406.8
(22) Date of filing: 20.12.2016
(51) Int. Cl.: A61C 17/06, A61C 19/00

(54) **DENTAL SUCTION ARRANGEMENT**
DENTALE SAUGANORDNUNG
SYSTÈME D'ASPIRATION DENTAIRE

(30) Priority: 23.06.2016 WO PCT/EP2016/064618
(43) Date of publication of application: 27.12.2017
(73) Proprietor: Asum, Thomas, 41136 Göteborg (SE)
(72) Inventor: Asum, Thomas, 41136 Göteborg (SE)
(74) Representative: Baldus, Oliver

(56) References cited:
- GB-A- 1 060 288
- US-A- 1 742 080
- US-A- 5 071 347
- US-A- 6 068 477
- US-A1- 2013 203 012

## Description

### Technical Field

The invention is about a dental suction arrangement which removes saliva and other fluids from a person's oral cavity. The suction arrangement includes a suction tube and an absorption body to be placed in the oral cavity, thereby the suction tube includes a suction part which is arranged in the absorption body and which is equipped with a hole member communicating with the absorption body.

### Technical background

In the dental field different types of suction arrangements are used for removal of saliva and water from the oral cavity. The supply of water mainly takes place when the tooth substance is drilled and grinded and when removing tartar for cooling the teeth using water spray in order to lower the teeth temperature and keep instruments work properly. Water supply is also common in connection with teeth and in the oral cavity for cleaning where water spray is often used. The removal of saliva and water is beneficial for several reasons, including patient's or user's comfort, working area visibility, dry keeping of an operating area, etc.

The removal of saliva and water is mainly performed using two kinds of suction devices including tubes with different dimensions, where the thicker one is a so called high-speed suction device that efficiently removes large volumes of water and saliva, and which is operated manually by the operator or his assistant. The suction device with a thinner dimension and lower suction capacity is mainly used in connection with a device which is fixed in the oral cavity and where the removal of saliva and water works automatically without impact of the operator. Often the suction device also works as a protection of the tongue against rotating instruments by keeping away the tongue.

One example of such a thinner dimensioned suction device is described in US 3,086,289. These kinds of suction arrangements consist solely of various kinds of plastic tubes, straight or pre-bent in different shapes, with holes placed in one end or along the tube. By reshaping the suction device by hand it is possibly to adjust the device to different locations in the oral cavity where the absorption at the moment it is most needed and where the suction device brings most benefit.

The problem is that all people look different in combination with the fact that the constructions are rather static with limited possibilities for adjustments of the absorption effect to areas where it is most needed. These constructions are neither able to absorb salvia and water further down at the bottom of the mouth adjacent to the Glandula Sublingualis and Glandular Submandibularis nor further back in the throat and they have only very limited ability to safe isolate an operating area from saliva since the absorption effect is limited only to the area where the suction device's holes are located, and then it only sucks air most of the time.

Another major problem is the low comfort of these constructions. The inside of the lower jaw is very sensitive to pain due to the fact that the bone only is covered with a thin pain-sensitive periosteum. This often results in a strong discomfort for the patient. To minimize this effect, now and then a need arises to adjust and change the shape of the suction arrangement, which interrupts the operator's work and can be very time-consuming.

When placing the suction device at the bottom of the mouth, the holes of the suction devices often get blocked since the mobile part of the mucous membrane is absorbed in to the holes and this leads to a total blocking of the hole or to a strong decrease of the absorption capacity of the suction devices. Additionally in such cases, often a loud and very disturbing noise arises when the absorbed mucous membrane vibrates in the holes of the suction devices.

From the past it is further known to provide plastic tubes with absorptions bodies and place these along each side of the tooth socket or along the row of teeth. Accordingly, some of the above described negative effects and problems connected with saliva absorption can be reduced. Examples of such types of suction devices are described in US 5,071,347 and US 6,309,218.

In order to efficiently absorb saliva and water from the oral cavity using such an absorption body it is important that the absorption body has good connection/coverage with the area where the saliva is produced and accumulated. The absorption body of the above mentioned known suction arrangement is cylindrical and placed along the tooth row in a static form. This results in an incomplete or missing connection with the respective area where the saliva is produced. Only removal of water and saliva that are hitting the cotton-rolls directly will take place. The area between the bodies are kept dry but the all the remaining fluid and saliva will reach the throat of the patient as if there has not been any suction device at all. These devices does not create any benefits for the patient and does not reduce the work needed from the dentist or assistant to keep the patient comfortable.

In US 2013/0203012 a siphoning device for surgery is described that has similar basic features but where the physical properties are very limited regarding dental use. The described device can not be placed in the desired area where it is most desired, between the tongue and the teeth row, in an easy way or in a secured fixed position. It is not protecting the tongue from a rotating instrument or a similar instrument at all.

US 5,071,347 discloses dental instruments for removing or ejecting saliva from the mouth of a patient during a dental procedure. The suction parts of the disclosed instruments (i.e., the parts of the suction line which are arranged in the absorption bodies) are not U-shaped or parabolic. Instead, the suction parts are straight elements disposed at opposing ends of two suction lines.

All common suction devices are today one-sided which are very insufficient when there is a need for keeping both sides dry i. e. during the cementation of a full-arch bridge in the lower jaw where the dry-keeping is essential. This is an operation of great stress for the dentist and assistant. With this solution the device could stay in place creating the needed dry operation field and eliminating the risk of saliva contamination. Using two ordinary devices would not help then they have to be removed before placing the bridge and then the risk for contminations is very high.

Another general problem with all different suction devices today are when there is time for i.e. checking and adjusting the heigh of a crown or a filling it is necessary for the patient to bite together on a occlusion-foil for having a colour-mark on occlusal bad spots. The suction device then has to be removed from the mouth. When the suction device is removed the saliva flows all over the teeth. The teeth have to be dried with the vacum-suction device before the patient is biting on the occlusion-folie. If the tooth surface is wet the colour from the occlusion-foil is not adhering so well to the tooth and the registration becomes false or unsure. The procedure with repeatedly drying is very time consuming.

Another dry-keeping problem occur when using modern 3D-laser scanning techniques for taking so called digital impressions of the teeth and teeth-rows. They have to be saliva-free or else there will be a defect impression not possible to use for producing the prosthetic constructions or similar. This is a problem especially with the lower jaw. When using today's suction devices where the suction tube is placed over the row of the teeth and it will interfere with the recording when scanning a full row in the lower jaw.

A general problem in dentistry is also the noise-level that is generally high due to air-driven high-speed turbines, scalers and the suction devices. Then the suction devices normally is in action all of the time, especially the "low-volume devices" described here, they are very tiring for the operators staying in this environment all day long.

Also when placed for a long time in the mouth the soft tissue in the mouth will become over-dried resulting in discomfort for the patient.

Thus there exists a need of an effective suction arrangement which removes the saliva and water where it is produced and accumulated, that it shields off the operation field bilaterally and keeps it dry, is comfortable for the patient by not creating pain or an over-dried mouth, should be possible to let stay in the mouth during biting or without interfering a 3D-scanning procedure and is an intelligent system communicating with the vacuum-producing system in creating a low-noise environment and saving energy.

The suggested device is anatomical designed for the space between the tongue and the teeth-row, where the saliva is produced by Glandula Sublingualis, Glandula Submandibularis, and in a more advanced version also Glandula Parotis, and has a secured fixation due the loop and the foam-body under the chin. Besides is has an extensive flexibility that adapts to a wide range of different sizes of patients.

### Summary of the invention

One object of the present invention is to remove problems associated with the prior art as mentioned above and to provide a more efficient absorption of saliva and other fluids while yet rationalizing the dental work flows.

The invention is defined by claim 1. Advantageous developments may be taken from the subclaims.

According to the invention, a suction line comprises a suction part, which runs through an absorption body. Hole members are provided in the suction part and saliva and other fluids are absorbed by the absorption body are sucked away through the hole members of the suction part.

According to the invention, the absorption body and the suction part are essentially U-shaped for covering the full arch of the lower jaw. The suction-line is connected to a vacuum source and the suction line exits from the absorption body at one end of the absorption body. The other end may be covered by the absorption body, may be provided with a hole or may be closed, depending on the needs. The absorption body and the suction part, in an advantageous development, are plastic.

At most favourable, the absorption body and the suction part are arranged orally close to the lower jaw. The suction line when exiting the absorption body is bent upwardly to pass over the lower jaw. This portion of the suction line, the transverse portion, is adapted to transverse the lower jaw at a teeth free area thereof, one sided or bilaterally. Following this transverse portion, the suction line is intended to run further exiting the mouth of the patient.

Thus, the dental suction arrangement according to the invention may be effective also during occlusion.

Having the suction device in place in the mouth gives great advantages as it is saving a lot of time in reducing time for dry-keeping because this procedure some times needs to be repeated several times before a perfect result is achieved. The teeth are kept dry and the colour from the occlusion-foils are easily attached to the dry surface. Also grinding the specific tooth is much more convenient when it is not covered with saliva or water.

With the suggested design the suction device will stay in place creating a dry scanning-field without any interfering parts from the suction device.

With the suggested design the noise is reduced but besides the physical properties reducing the noise-level a built-in moisture-sensor or flow meter is suggested. The moisture-sensor could be placed in the foam-body or on the suction-tube. Preferable of a wireless type sending information to the suction system to reduce the "power" needed in the system. This will lower the surrounding tiring noise level and reduce the fatigue feelings created by the high noise levels then the system will only operate on high levels when needed.

Through the elastically deformable material, the main part of the outer surface of the suction arrangement will be connected with areas at the bottom of the mouth from where the fluid will be absorbed. This enables a more effectively and completely absorption.

Also, it is important that the suction line with the absorption body extends not only to the lower part of the lower jaw but that it extends along the lower part of the lower jaw. By this, the absorption body is sort of clamped between the tongue and the lower jaw, such that it will not unintentionally disengage from this place.

This is especially important when the patient swallows as with the prior art devices, due to intense movements of the oral cavity, the suction tube usually then got lost.

The suction arrangement, which comprises elastically deformable material, adapts to the bottom of the mouth and covers the area without causing any discomfort for the patient and at the same time preventing the saliva to come in contact with the teeth, and further the device is retained at the bottom of the mouth since it is partially placed under the tongue.

It may extend at least partially under the tongue, thus, forming a partial U-shape. Preferably, however, the absorption body and the suction part have an essential U-shape following the oral side of the lower jaw.

The suction line is preferably made of plastic material. The absorption body may be fixed the the suction part of the suction line in any suitable manner, such as by glueing or by friction. The suction arrangement is suitable both for to be applied to the left or the right lower jaw.

The absorption body within the suction arrangement also works as a pump when it is compressed due the movement when the patient swallows and the subsequently expansion. In order to ensure the desired and beneficial pump action, it it essential for the elastic absorption body to have a compressibility to half of its original volume by standard tongue force. Thus, the material of the absorption body may be soft open foam with a low elasticity coefficient, or a soft flocking, or a silky mesh covering the inner absorption body..

The absorption body also eliminates the need to interrupt e.g. the ongoing dental treatment for adjustment of the suction device when it causes painful pressure to the patient's inside of the lower jaw.

Due to the fact that the suction arrangement is anatomical preshaped it adapts itself to the anatomical shape of the bottom of the mouth and also is highly elastic, it automatically very efficiently adapts to the exact anatomical shape in a way which is not possible with a simple cylindrical absorption bodies under known technique. Also the dental treatment will be more silent since the absorption body acts as noise shield, dampening the suction noise emitted by suction holes. The created anatomical form effectively allow the reception of saliva leaving both Glandula Sublingualis and Glandula Submandubularis, and the device builds a barrier between the teeth and the saliva glands. In an additional design there is an absorption body also covering the ductus of Glandular Parotis.

In one embodiment of the invention, the absorption body shows, in a cross sectionional view, perpendicular to the absorption body's length extension, a non circular contour.

In another embodiment, the absorption body shows, in a in a cross sectionional view perpendicular to the absorption body's length extension, a contour with a base and a top, where the mentioned base is broader than the mentioned top. This shape facilitates the placement and retention under a part of the tongue. In another embodiment, the absorption body has a part, which, in a cross sectional view perpendicular to the absorption body's length extension, shows a contour with at least one concave part. With the concave contour part the shape of the absorption body is adapted in a natural way in order to create space for the tongue. This will avoid concentration of pressure at the tongue and at the same time the absorption body effectively reaches areas, over as well as under the tongue, where the absorption is most needed.

Preferably, the cross section of the absorption body is larger below the area of the waist which is created by the tongue and slightly smaller above this waist. The suction part of the suction tube' is preferably arranged at the lower, broader part. In one embodiment, the suction tube is made of elastically deformable and adjustable material in order to obtain an adaption to the anatomical shape of the bottom of the mouth and placement position. This embodiment includes a variety of application possibilities. The suction device and its related suction tube or suction line and absorption body especially can adapt itself to different sizes of the bottom of the mouth, but also since the absorption body is so long it has the possibility to extend along the alveolar area or gingival area of the jaw at the incisors and canines as well as at premolars and molars, and due to the cross sectional shape it adapts to the different parts.

In a further embodiment, the absorption body is adjustable in its length. A long absorption body will be prefabricated, and it will be shortened by cutting it. Alternatively, the absorption body may be fixed by friction on the suction part of the suction line, and be manually extended and shifted according to the needs. Further alternatively, the suction part may be provided with a punch pattern, allowing its extension together with the absorption body. Thus, it is advantageous to have the U-shaped absorption body and U-shaped suction part at a suitable lengths which would fit into the largest jaws. When tweeting a patient with a smaller jaw, the suction part and the absorption body is shortened, i. e. by cutting them off. The free end hole of the suction part may be closed in any suitable manner.

In a further embodiment, the hole member comprises multiple holes. This facilitates the transport of fluid from the outer surface of the absorption body to the absorption part or suction part since the transport routes become shorter. Preferably, the multiple holes are distributed along the absorption part as well at its circumference.

In a further embodiment, the holes are of different size and/or shape. The hole size and/or shape or both can thus be adjusted to local variations in relation to the need of absorption at the different points of the absorption body. The need of absorption may vary depending on where the saliva is produced, on locations where saliva tends to accumulate, on locations where other fluids accumulate and on variations in the absorption body's deformation. Also from a flow technical point of view it is favorable to be able to have different sized holes depending on their distance to the connection of the absorptions part to the rest of the suction device.

In a further embodiment, the suction device includes multiple absorption bodies and the suction tube includes additional lines, whereby each additional line is connected with a suction part in the respective absorption body.

In a further embodiment, the respective suction part is detachably connected with the rest of the suction line.

This allows an easy replacement of absorption bodies in case of some reason the absorption capacity would decline. This also allows that one and the same suction tube can be connected to different types of absorption bodies.

In a further embodiment, the suction device includes a suction line and a set of multiple absorption bodies in different embodiments in respect of the anatomical shape and/or size.

With such a set a high flexibility is achieved, since a single suction line can be connected to any type of absorption bodies depending on the current situation. Alternatively, it allows having a set of one absorption body-type in different sizes to choose between for an optimal fitting depending on the patient's jaw size or other patient dependent variables.

In a further embodiment, the suction device includes a protection shield which may be a tongue protection in order to block the tongue. The tongue protection aims to protect the tongue from rotating instruments during the dental treatment such as cleaning treatment, and also to prevent the tongue from touching the tooth or teeth in critical areas which may cause a contamination with saliva when performing different filling and/or cementation of prosthetic constructions.

in one embodiment, the tongue protection is arranged at an extension of the suction line, and in another embodiment, the tongue protection is arranged at one sidearm of the mentioned suction line.

In a further embodiment, the tongue protection is made of a material which is elastically deformable. With this embodiment, the advantage is obtained that the tongue protection can be bent in order to effectively separate the tongue from the jaw area, while also allowing the assisting personal to obtain full access and insight in any critical area for required interventions and complementary removal of water and saliva.

in one embodiment, the tongue protection is made of a material which can be broken off in order to adjust the fitting and placement in the oral cavity and also in order to enable an increased access and insight, while the ability to protect the tongue is retained.

In a further embodiment, the tongue protection is arranged adjustably to the suction tube. This is an additional opportunity to perform specific adjustments depending on the condition of the patient.

In a further embodiment, the tongue protection has a absorption capacity. This absorption capacity can either be provided in case the tongue protection is made of absorbable material, or covered by absorption material or in case the tongue protection is equipped with a - secondary - suction line which is connected with the suction device and its absorption body.

In one embodiment, the respective tongue protection and suction part are detachable connected with the rest of the suction tube.

The secondary suction line between the tongue protection and the absorption body may be such that it is bias to separate the tongue from the lower part of the lower jaw. By this, the absorption body is pressed downward towards the Glandulae under the tongue. The secondary suction line advantageously is bent by about a little less than 180 degree.

in another embodiment of the invention, the tongue holder may be provided with a grip, like a small lateral extension. By this any operator like a dentist may pull the tongue holder sidewards or in any suitable direction.

in another embodiment of the invention, the tongue holder is turnable and/or attached via a plastic hinge to the secondary suction line.

in another embodiment of the invention, the tongue holder is made from thermosetting material. The operator then may heat it before use, and shape it to any desirable form. After re-cooling it will maintain the selected form. This process may be repeated frequently if desired.

The tongue holder advantageously may be made from orange acrylic material to form a light curing shield.

The suction line may be made of any suitable material such as special plastic material. The material may be selected to have a memory effect.

In one embodiment, the suction device includes a suction tube or suction line and a set of multiple absorption bodies in different executions in respect of the anatomical shape and/or size, and a set of multiple tongue protections in different executions in respect of the anatomical shape and/or size.

In a very advantageous embodiment of the invention, the absorption body is covered be a silky mesh, such as known from jewelry boxes. Such a web or mesh has a surface with a lowfriction coefficient such such the absorption body may be inserted easily to its desired place. It has a very pleasant and comfortable feeling for the patient. The silky mesh is preferably placed laterally, on the sides of the absorption body, against the mucous membrane of the jaw and the side of the tongue and is of a non-permeable material in this case for reducing the vacuum-force here.

Preferably, the silky mesh is very thin and thus very flexible. Also, the absorption body is soft and flexible and has an elastic character. If it is made from foam or any other suitable material, a hardness gradient may be used such that it is harder close to the suction part and softer close to its outer periphery.

Such a hardness gradient automatically is provided if the absorption body is made of a flocking. Any desired shape and distribution is possible. Flocking has a very good capillary effect.

In another embodiment, the suction arrangement is provided with a moisture sensor, intended to give a feedback to the vacuum pump and to reduce the pump action if the area under the tongue is dry. This will also reduce the vacuum pump noise.

The inventive suction arrangement is also very suitable for toothless patients. Because of the spring action both to the absorption body between the tongue and the bottom of the mouth, and of the suction line between the chin and the lower part of the lower jaw. The suction line acts as sort of a clamp pressing the suction portion downwards by interaction with the exposed part of the suction line which is bent such that it is pressed against the chin. The invention works great independently of whether the jaws are fully teethed, partially teethed or unteethed.

Other purposes, features and facilities of the invention will be shown in the following detailed description, in the patent claims, as well as in the drawings. It ought to be understood that additional advantageous embodiments may arise via different possible combination of the features from the described embodiments and with each possible combination of these features described in the examples following presentation.

Generally, the terms in the requirements should be interpreted in accordance with their normal meaning in the technical field, unless explicitly stated otherwise. All references to "a / an / the / [a suction arrangement, a suction tube, an absorption, etc.] " should be interpreted openly as a reference to the existence of at least one mentioned suction device, suction tube, absorption body etc. unless explicitly stated otherwise.

### Brief description of the drawings attached

These and other embodiments of the invention will now be described in more detail with reference to the attached drawings, which show embodiments of the invention.
Fig. 1 is a perspective view of a suction device or suction arrangement according to a first embodiment of the invention;
Fig. 2a and 2b are other embodiments of the suction device or suction arrangement according to a second and third embodiment of the invention;
Fig. 3 is a sectional view along line III in Fig. 2A;
Fig. 4 is a perspective view of another embodiment of the invention;
Fig. 5 is a perspective view of another embodiment of the invention;
Fig. 6 is the embodiment according to Fig. 5 in a cross-sectional view;
Fig. 7 is a perspective view of another embodiment of the present invention;
Fig. 8 is a perspective view of another embodiment of the present invention;
Fig. 9 is a perspective view of another embodiment of the present invention;
Fig. 10 is a perspective view of another embodiment of the present invention;
Fig. 11A is a perspective view of another embodiment of the present invention;
Fig. 11B is a perspective view of another embodiment of the present invention showing the extension parts of the U-shaped body;
Fig. 12A is a perspective view of another embodiment of the present invention with built in moisture sensors/flow meters with wire connection;
Fig. 12B is a perspective view of another embodiment of the present invention with built in moisture sensors/flow meters with wire-less transmitting;
Fig. 13 is a perspective view of another embodiment of the present invention;
Fig. 14 is a sectional view of another embodiment of the suction arrangement of the present invention, with a modified absorption;
Fig. 15 is still another embodiment of the suction arrangement according to the invention; and
Fig. 16 is a perspective view of another embodiment of the present invention;
Fig. 17 is a perspective view of another embodiment of the present invention;
Fig. 18 a partial sectional view according to Fig. 17; and
Fig. 19 is a sectional view of another embodiment of the present invention.

### Description of embodiments

The invention will now be described in more detail with references to the attached drawings.

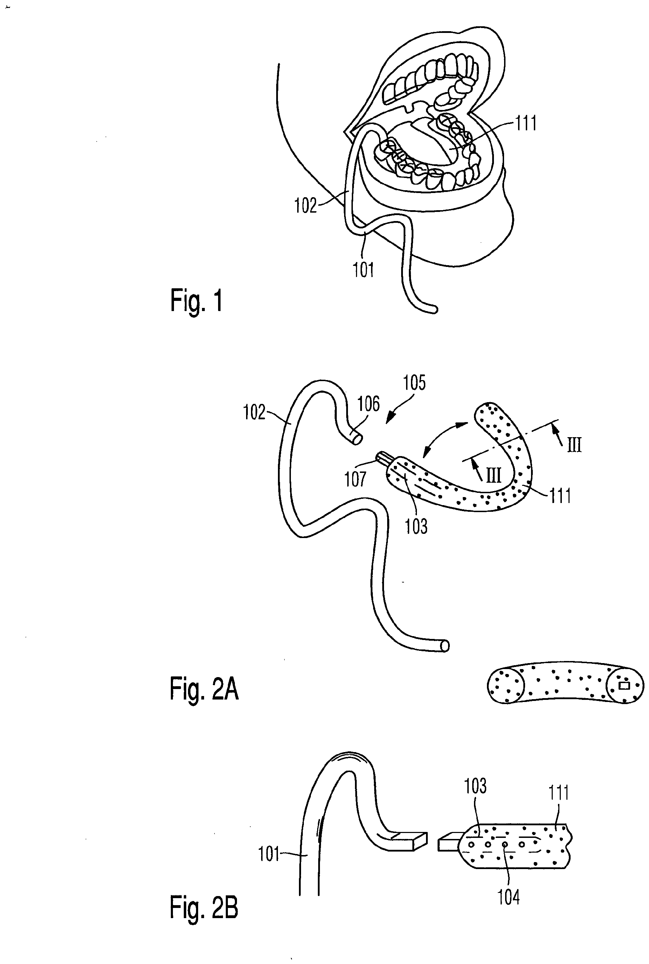

in Fig. 1 , a first embodiment of the invention is illustrated. As shown in Fig. 1, the suction device has a suction tube or suction line 101 which can be connected to a vacuum source in order to create a negative pressure inside the suction tube. The suction tube 101 has an exposed part 102 which is visible. The suction tube 101 extends into the absorption body 111 of the suction device with a suction part 103 embedded in the absorption body (not visible in Fig. 1). The absorption body 111 which surrounds the suction part 103 is arranged to be placed at the user's bottom of the mouth between the lower part of the lower jaw and the tongue.

The absorption body 111 thus follows the oral side of the dental arch. The absorption body is essentially U-shaped, or, to be more specific, parabolic. The form of the absorption body 111 is mainly determined by being adjacent or close-fitting to the lower part of the lower jaw. On the other hand, the suction part 103 extends through the absorption body 111. The suction part 103 has a greater stiffness than the absorption body 111 which absorption body 111 comprises preferably an open porous foam which, in a advantageous development, is covered by a silky mesh. The suction tube or suction line 101 which also comprises the suction part 103 is made from deformable plastic, and the suction part 103 comprises holes through which saliva or similar fluids may be sucked away.

In this example, the exposed part 102 of the suction tube 101 is shown . The suction part 103 may alternatively be joined to a juncture 105 between the suction part 103 and the exposed part 102. This is shown in Fig. 2a and 2b. The junction 105 comprises a sleeve 106 formed on the exposed part 102 and a plug 107 formed on the suction part 103.

The suction part 103 is equipped with multiple holes 104 which connect the tubes inner part with the covering absorption body. In this example, the absorptions body is arch-shaped or parabolic shaped, but since the suction device is made of elastic deformable material the device is adapted to fit into the patient's oral cavity and the bottom of the mouth in particular.

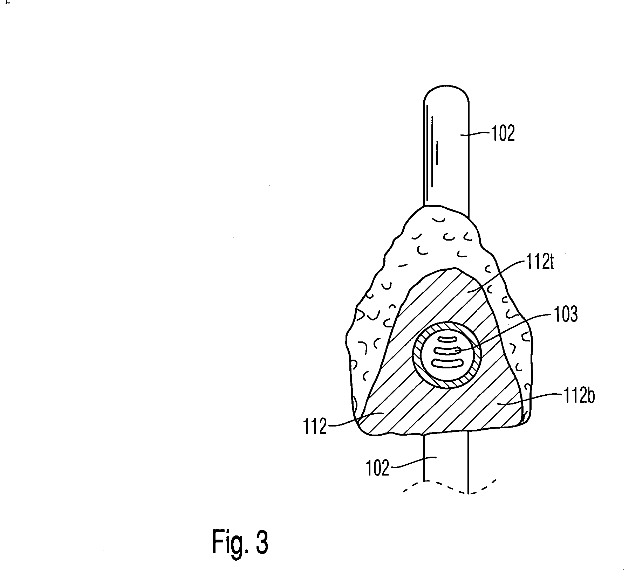

Fig. 3, which is a sectional view along line III-III in Fig. 2a, shows that the absorption body in the lateral direction has a non-circular outer contour with a base 112b and a top 112t, whereby the base 112b is broader than the top 112t, and shows a triangular-like cross section. The suction part 103 of the suction tube 101 is centrally located in the absorption body. Alternatively to the illustration, this part can be located in other positions in the absorption body, e.g. in an asymmetric placement. Also, the outer contour may be different than the exemplified.

The suction line 101 is connected to a vacuum source which is not shown. Based on this vacuum, there is a fluid flow through the suction device 101. Saliva and water enter the absorption body 111 from mucous membranes and from other places in the oral cavity, where the fluid accumulates. The suction tube 101 is connected to vacuum whereby underpressure is generated inside its suction part 103. By capillary action the fluid entering the absorption body 111 is absorbed in the direction of the suction part 103. Through the vacuum the fluid is absorbed by the suction tube, and is removed.

Since the suction device 101 is made of elastic deformable and adjustable material, the suction device can be bent and be placed at the bottom of the mouth beside and along the lower part of the lower jaw by the incisors and extend back toward the lower part of the lower jaw by the premolars and molars on the right and/or left side.

The suction tube 101 is preferably made of extruded plastic and if needed also includes an integrated wire that allows the operator to reshape the tube if necessary. The suction tube has prefabricated shape and size that allows it to be easily placed at the bottom of the mouth and at the same time to cover the lower part of the lower jaw for a secure fixation.

The suction tube may have - in a cross-sectional view - a round shape or an oval shape which is even more easy to bend. Also, the wire may be flat, having a softer spring characteristic in its flat direction. Both may be adapted to the user's needs.

The suction part 103 of the suction tube can, be provided with holes 104 of different size and shape. Some holes are circular with different diameter where the holes 104 closest to the exposed part of the suction tube or line are portion are the smallest. Other holes are elongated.

The absorption body 111 is made of suitably elastically deformable material which enables fluid transport through the body, preferably foam plastic with open cells. It is also possible to use other soft materials that have capillary action like for example, cotton fibers, various textile fibers, synthetic fibers, mull, lint or gauze, preferably covered by a mesh, and the like.

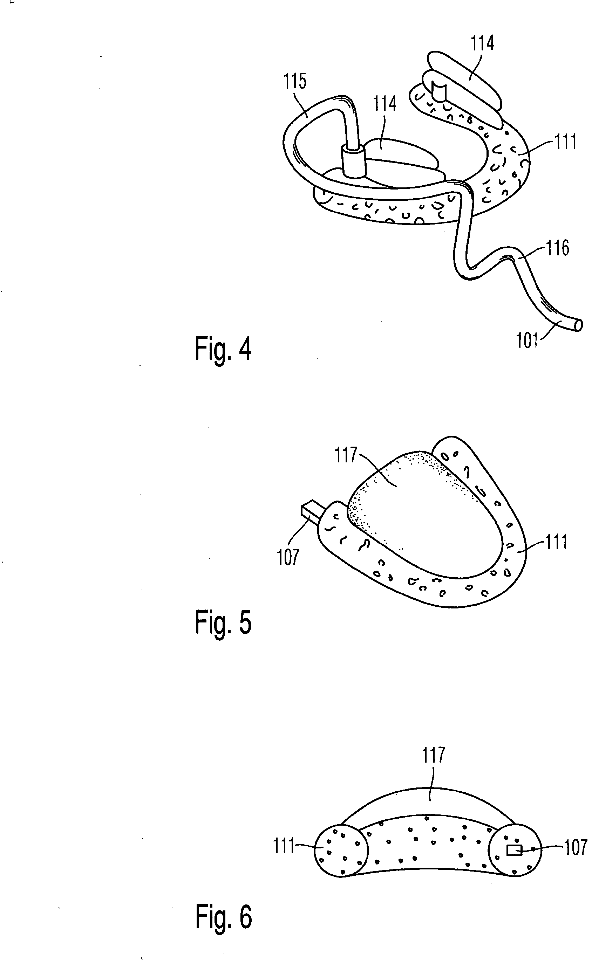

In the embodiment example illustrated in Fig. 4, the suction device comprises a tongue protection 114. The tongue protection aims to block the tongue and so protect the tongue from rotating instruments during the dental treatment and also to prevent the tongue from touching the tooth or teeth in any critical area which may cause a contamination with saliva when performing different filling and/or cementation of prosthetic constructions. Fig. 4 illustrates an embodiment, where two tongue protections 114 are arranged on both end sides of the absorption body 111. In this embodiment they are connected to the suction part 103 and extend through the foam of the absorption body 111 , but in another embodiment, the tongue protection 114 may be arranged on one sidearm of an exposed suction tube 120. In the present embodiment, the suction device 101 includes two tongue protections, one arranged on one sidearm 120 an one arranged on the exposed suction tube 102. Two tongue protections can be made of elastically deformable material. Thus, the tongue protection can be bent, and therefore effectively separate the tongue from the operation, while also allowing the assisting personal to obtain full access and insight for required interventions and complementary removal of water and saliva.

In one embodiment, the tongue protection is made of a material which can be broken off, in order to adjust the fitting and placement in the oral cavity and also in order to enable an increased access and insight while the ability to protect the tongue during treatment is retained.

Furthermore, the tongue protection may be arranged in relation to the suction part 103 of the suction tube 101 and the absorption body 111.

In a further (not illustrated) embodiment, the tongue protection has an absorption capacity. This absorption capacity can either be provided in case the tongue protection is made of absorbable material or when the tongue protection is equipped with a suction tube which is connected with the suction device and its absorption body.

As may be taken from Fig. 4 (and Fig. 1), the suction tube 101 or suction line 101 extends upwardly from the absorption body 111. It is intended to transverse the lower jaw distal from the last molar, there crossing a tooth-free and gingival portion of the lower jaw. This part is designated as transverse part 115 of the suction tube 101. The suction line 101 then enters the user's mouth downwardly along the user's chin. The suction line comprises a counter bow 116 which is intended to be pressed against the user's chin from its lower side such as to keep the absorption body 111 in place.

Another embodiment is shown in Fig. 5. It has a plug 107 which is intended to be attached to a sleeve 106 of the exposed part 102 of a suction line 101.

The absorption body 111 is essentially parabolic or essentially U-shaped. Contrary to the remaining embodiments, this embodiment comprises a tongue shield 117 which is intended to keep the tongue at its regular position and to avoid tongue play which would unduly disturb surgery by the dentist.

The tongue shield 117 may be made of any suitable material and also may be very elastic. The tongue shield may be attached to the absorption body 111 but also to the suction part 103 running through the absorption body 111.

A back view, i. e. a distal view, of this embodiment is shown in Fig. 6.

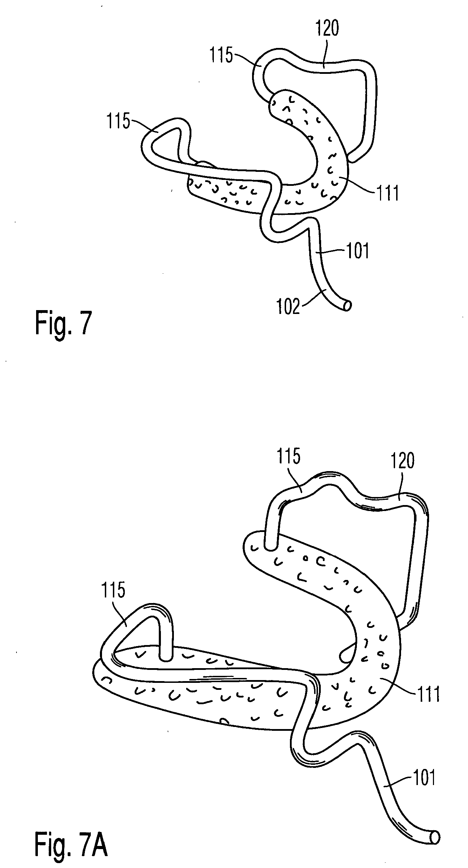

Fig. 7 shows another embodiment comprising a U-shaped or parabolic shaped absorption body 111 and a suction line 101, with its exposed part 102 being shown.

Contrary to other embodiments, there is a exposed suction tube 120 extending from the other hand of the absorption body 111. This exposed part is intended to also comprise a transverse part 115. The exposed part 120 is shaped in a suitable manner to keep the absorption body 111 in place by bi-lateral fixation under the chin. The suction line 101 exiting from the mouth may have any suitable form.

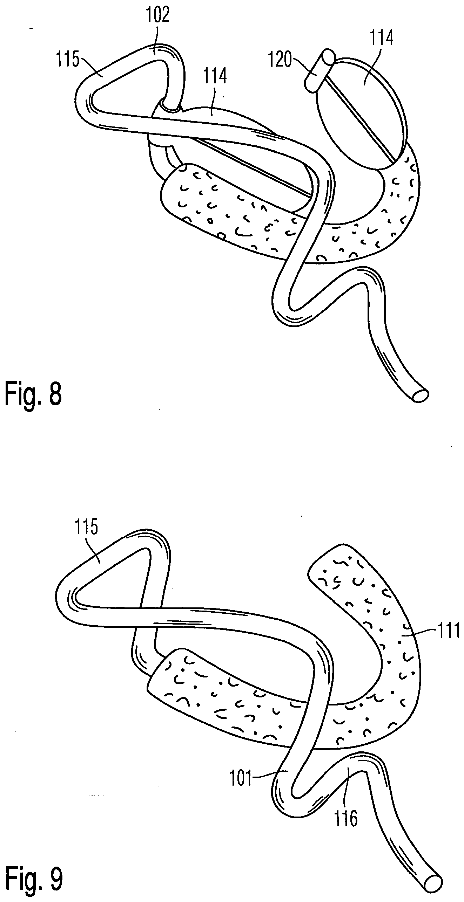

Another example is shown in Fig. 9. With this example, the counter bow 116 is intended to be arranged centrally under the user's chin, thus to make the downward pressing force of the suction line towards the absorption body 111 having a less unilateral attack angle.

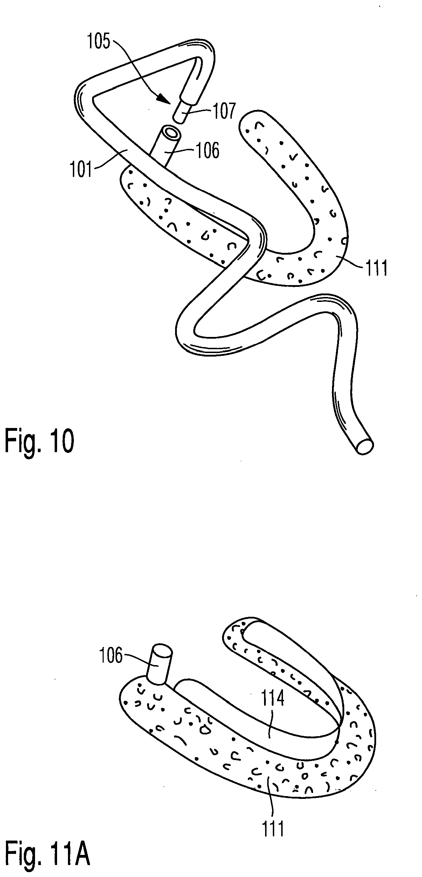

A similar arrangement may be taken from Fig. 10. In this arrangement, a junction 105 comprises with a sleeve 106 and a plug 107. Contrary to Fig. 2a and 2b, the sleeve 106 with this embodiment is arranged close to the absorption body 111.

In Fig. 11A, another embodiment of an absorption body 111 is shown. This embodiment comprises a tongue shield 114 which extends upwardly from the absorption body 111 and has a parabolic shape intended to keep away the tongue from the dental arch.

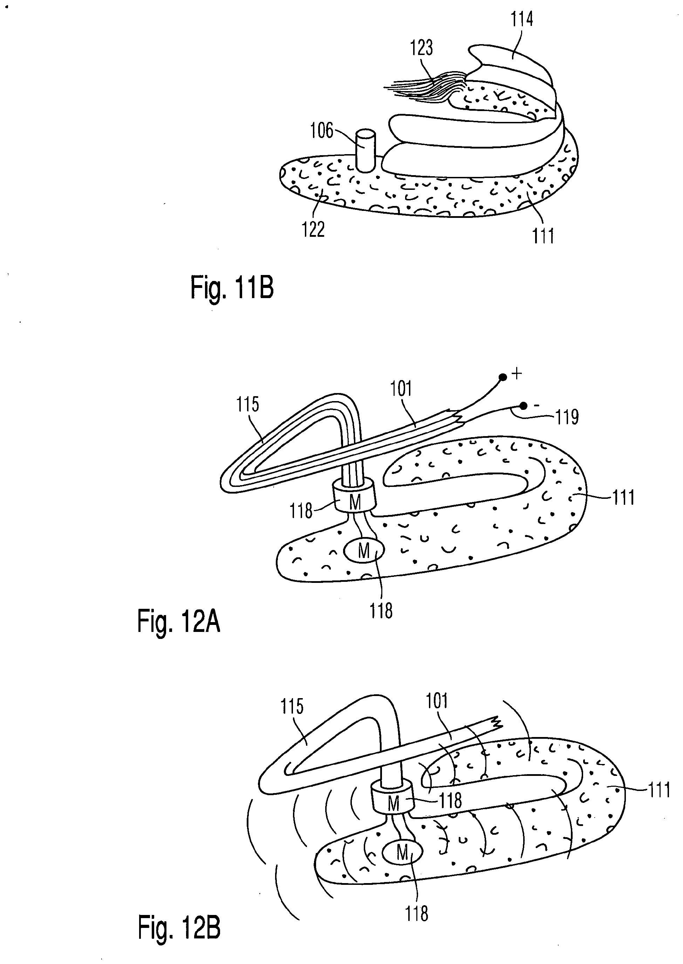

in Fig. 11B, the same as 11A but with the extension parts shown 122 as a part of the foam body and 123 as a separate connected part of mesh or fibers or similar.

Advantageously, at least one protection shield is used which is attached to the absorption body 111, the suction part 103 and/or the exposed part 102 of the suction line 101, or a free end thereof. The protection shield 114 or the protection shields 114 may be bendable and can be brought into any desired form. They may have at least one breaking line, and/or may be light cure shields.

Fig. 12A shows another embodiment which comprises a moisture sensor 118 which is attached to above absorption body 111 or mounted within the absorption body 111 which is shown schematically in Fig. 12. There are supply lines 119 running through the suction line 101 for electrical connection of the moisture sensor 118.

Fig. 12B shows another embodiment which comprises a moisture sensor 118 which is attached to above absorption body 111 or mounted within the absorption body 111 which is shown schematically in Fig. 12B. These sensors are transmitting wireless to a suitable device, e.g. the vacuum-system.

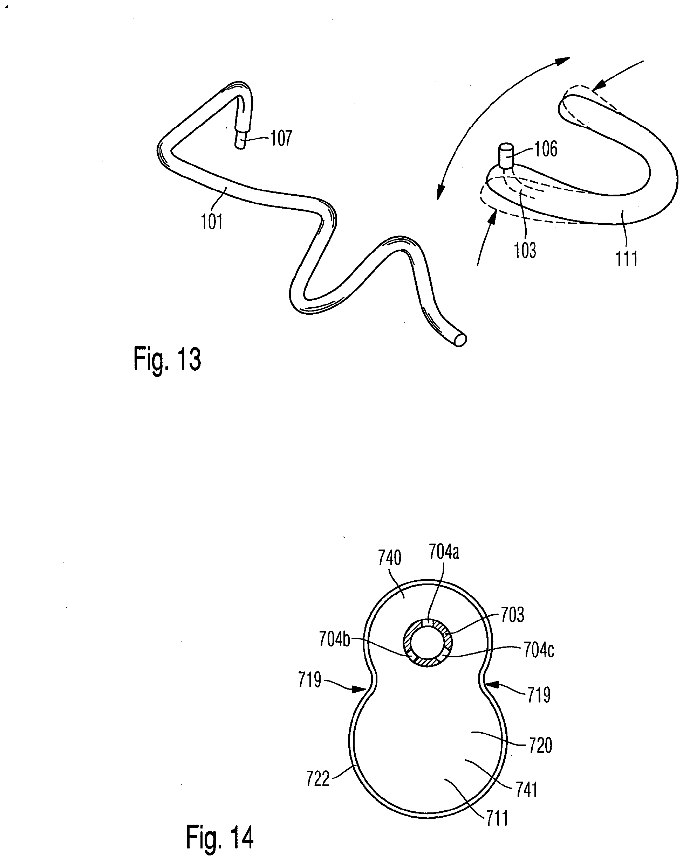

From Fig. 13, it may be taken that the absorption body 111 comprising the suction part 103 may be bent to any suitable form, within the anatomy as desired.

If needed it may be also be shorted by cutting off its ends or at least one end thereof.

The absorption body 111 is made by open-porous foam with a high elasticity, i. e. a soft open foam. This foam, according to the shown embodiment, is covered by a silky mesh which is also open-porous, to let the fluid pass, and is pleasant and comfortable to the patient. Alternatively, it is closed-porous to block fluids.

Fig. 14 shows another embodiment of the inventive dental suction arrangement.

A suction part 703 passes through the top portion 740. It has a plurality of suction holes 704a, 704b and 704c which are distributed uneven and with different diameters and form all over the suction part 703.

Preferably, the suction part 703 extends in the centre of the top portion 740. In the different embodiment it may extend eccentrically, in order to increase the suction power at one side of the absorption body 711.

The idea is to have the absorption body 711 being arranged as low as possible under the patient's tongue in the bottom of the mouth, close to Glandula Submandibularis and

Glandula Sublingualis, and a tongue holder extending upwardly from there and pressing the tongue obliquely upwardly and way from the jaw.

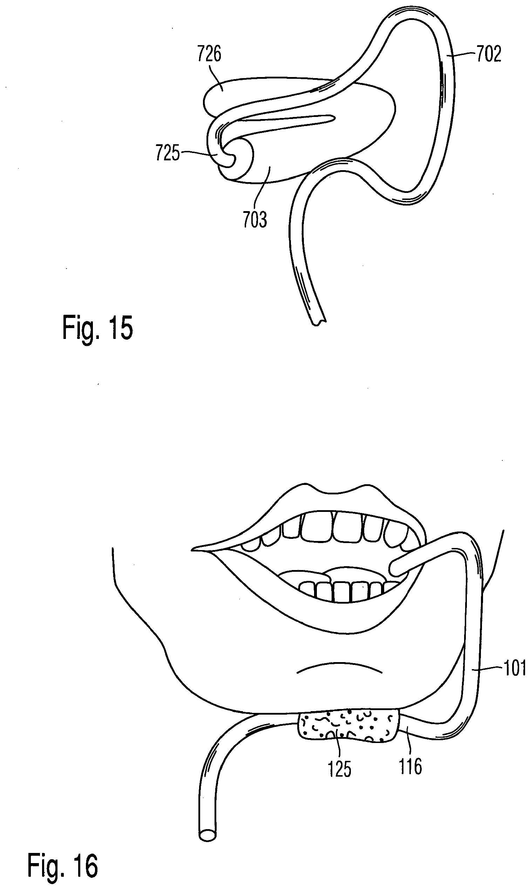

Another embodiment is shown in Fig. 15. This embodiment comprises a suction line 702 and a so-called "full jaw" absorption body 703. This absorption body is essentially U-shaped and comprises a suction part with suction holes even this is not shown in Fig. 15. It is intended to be placed under the tongue and the suction line 702 is of a flexibility such that my be bended at a hinge area 725, to have this essentially U-shaped absorption body being inserted into the oral cavity either such that the suction line 702 is arranged on the left side or on the right side.

According to the invention, the absorption body 111 is placed under the tongue, following the essential parabolic shape of the dental arch at the oral side. The absorption foam body is of a open porous foam such that it may be compressed between the tongue and the dental arch to up to 85 volume percent. A transverse region 115 of the suction line 101 is provided which extends essentially horizontal for crossing the jaw after the last molar on one side of the mouth or on both sides of the mouth. The transverse portion 115 is caught between upper and lower jaw, thus indirectly fixing the absorption body 111. The absorption body 111 is fixed especially in occlusion but is pressed downward even before occlusion by the flexible suction line 101.

For further improving the fixture, a counter bow 116 is provided in the suction line 101 which is intended to further fix it under the user's chin.

According to Fig. 16, this counter bow 116 may be provided with an additional foam body 115 for improving user's comfort and increasing the retention due to a higher friction against the skin and at the same time a larger contact area.

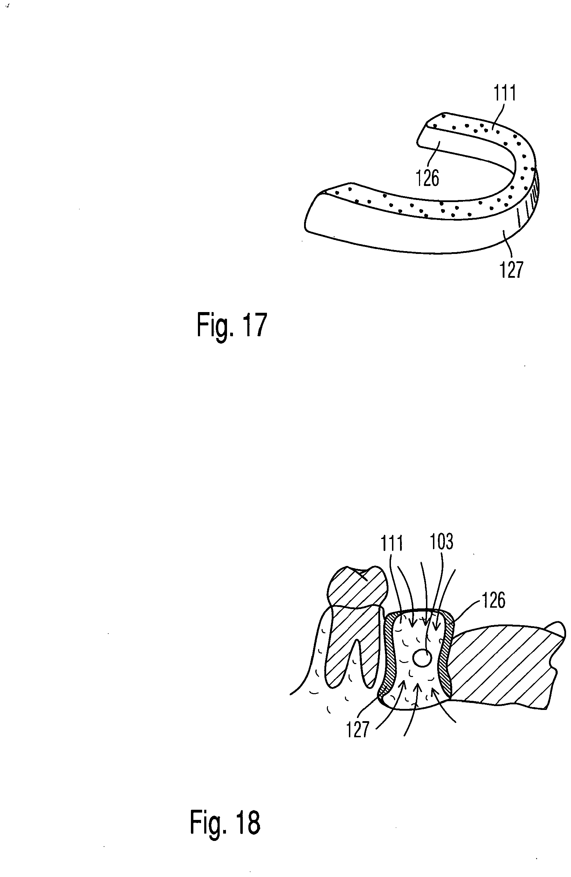

A further embodiment of the absorption body 111 is shown in Fig. 17 and 18. In this embodiment, the absorption body is provided with a silky mesh 126 and 127 on both the vestibular side and the oral side .

Thus, as may be taken from Fig. 18, the suction force is focused both on the upper side and the lower side of the absorption body 111 which is beneficial to improve suction efficiency.

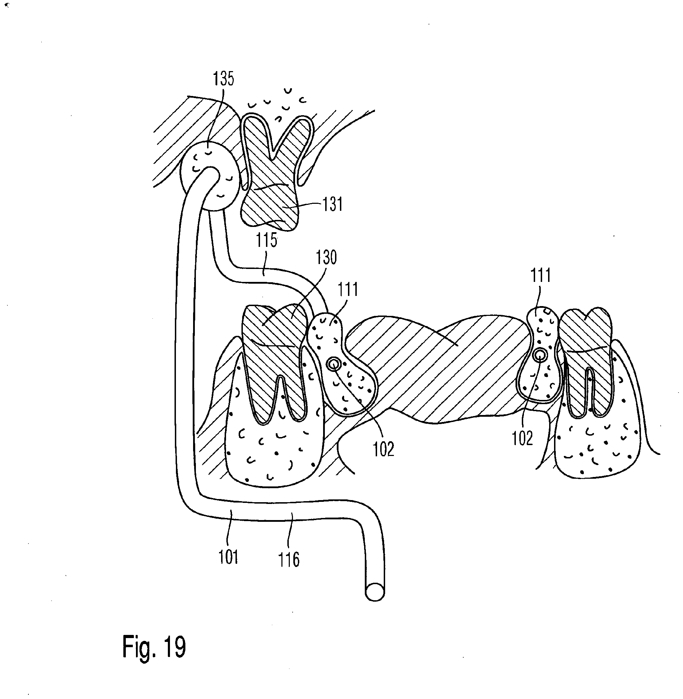

Another embodiment of the invention is shown in Fig. 19. This embodiment is shown, for the purpose of clarity, before occlusion, i. e. with antagonists still being separate from another. Yet it is intended to be functional in occlusion, with the transverse portion 115 extending distal from or behind the last molar 130.

The suction line 101, when exiting the transverse portion 115, is bent upwardly and is provided with an additional foam body 135 which is intended to be arranged vestibular of the antagonist 131. From this area, the suction line 101 runs downwardly and form a counter bow 116 as shown in other embodiments.

The invention has been described in relation to the current understanding of which are the most practical and preferred embodiments, but it is recognized that the invention is not limited to the described embodiments; several variations and modifications are possible. The scope of the invention is therefore exclusively defined by the attached patent claims.

## Claims

1. Dental suction arrangement for the removal of saliva and other fluids, where the suction arrangement includes a suction line (101) and an absorption body (111) to be placed in the oral cavity of a person for the removal of saliva and other fluids, wherein the suction line (101) includes a suction part (103) which is arranged in the absorption body (111) and which is equipped with hole members (104) communicating with the absorption body (111), wherein the absorption body (111) and the suction line (101) running through it, i.e. the suction part (103), are essentially U-shaped and/or parabolic shaped,
the suction line (101) exits from the absorption body (111) at one end thereof, **characterised in that** the suction line (101) has prefabricated shape and size that allows it to be easily placed at the bottom of the mouth and at the same time to cover the lower part of the lower jaw for a secure fixation, and **in that** the suction part (103) extends along the lower part of the lower jaw so as to be clamped between the tongue and the lower jaw of the person..

2. Dental suction arrangement according to claim 1, **characterized in that** a transverse portion (115) is formed in the suction line (101), following the U-shaped suction part (103), said transverse portion (115) being suitable for crossing a jaw distal of the row of teeth.

3. Dental suction arrangement according to claim 1, **characterized in that** the suction line (101) has a flattened cross section at a transverse portion (115) and preferably an essentially circular cross section elsewhere.

4. Dental suction arrangement according to one of the preceding claims, **characterized in that** the suction line (101) is deformable for adapting it to an anatomy, and preferably that said suction part (103) forming an U is deformable **in that** at least one side leg of the U may be bent into the desired shape.

5. Dental suction arrangement according to one of the preceding claims, **characterized in that** at least a tongue holder and/or a tongue shield (114) or protection shield (114) is attached to at least one portion of said suction line (101) or the suction part (103) or the absorption body (111).

6. Dental suction arrangement according to one of the preceding claims, **characterized in that** the absorption body (111), in a cross sectional view perpendicular to the longitudinal extent of the absorption body (111), has a non-circular contour and/or has a contour with a base and a top, wherein the mentioned base is broader than the mentioned top, and/or has a contour with at least one concave part.

7. Dental suction arrangement according to any of the previous claims, **characterized in that** the absorption body (111) includes an extension part which is a part of the absorption body (111) or a separate part made from a flocking material or fibers, said extension part preferably when placed in the cavity, extends distal from the suction line (101), wherein the extension part preferably has a reduced cross-sectional area which decreases in the direction of its distal end.

8. Dental suction arrangement according to any of the previous claims, **characterized in that** the absorption body (111) is adjustable in its length, preferably by being ductily deformed, and/or has a base body with a punch pattern allowing said body together with a foam body of the absorption body to be extended.

9. Dental suction arrangement according to any of the previous claims, **characterized in that** it includes a secondary suction line (101) made of adjustable material extending from the main absorption body (111) for connection with a secondary absorption body (111) and/or that the suction arrangement comprises multiple absorption bodies and that the suction line comprises additional lines, whereby each additional line is connected with a suction part in the respective absorption body, preferably for suction at the Glandular Parotis, at one side thereof or at both sides thereof.

10. Dental suction arrangement according to any of the previous claims, **characterized in that** it further includes at least one protection shield (114) for withholding a third part from moving toward any part of the dental suction arrangement, preferably being adjustable in shape and/or size.

11. Dental suction arrangement according to any of the claims 6 to 10, **characterized in that** said cavity is an oral cavity, an elevated portion is a lower jaw, said protection shield is a tongue protection and/or said secondary absorption body is arranged for being positioned at the parotis gland duct and/or the bottom being the area between the tongue and the bottom of the mouth.

12. Dental suction arrangement according to any of the previous claims, **characterized in that** a foam body is placed along the suction line (101) on the counter bow (116), paricularly for being fixed outside the mouth under the chin, and/or that both ends of the suction line (101,120) form counter bows (116), paricularly for extending under the chin for double-side fixation of the U-shaped and/or parabolic shaped absorption body (111).

13. Dental suction arrangement according to any of the previous claims, **characterized in that** a metal wire or another stiffening element extends within the suction line (101) or at least within the outer circumference of the suction line (101), for improving a possible adjustment of the shape of the suction line (101) and/or that a moisture sensor (118) or a flow meter (118) is provided at the suction line (101) or the absorption body (111) which is supplied with a wire (119) or is provided as a wire-less device.

## Patentansprüche

1. Dentale Absauganordnung zum Entfernen von Speichel und anderen Fluiden, wobei die Absauganordnung eine Saugleitung (101) und einen Absorptionskörper (111) zum Platzieren in der Mundhöhle einer Person zum Entfernen von Speichel und anderen Fluiden umfasst, wobei die Saugleitung (101) einen Saugteil (103) umfasst, der im Absorptionskörper (111) angeordnet ist und der mit mit dem Absorptionskörper (111) kommunizierenden Lochelementen (104) ausgestattet ist, wobei der Absorptionskörper (111) und die durch ihn verlaufende Saugleitung (101), d.h. der Saugteil (103), im Wesentlichen U-förmig und/oder parabelförmig ist, wobei die Saugleitung (101) den Absorptionskörper (111) an einem Ende davon verlässt, **dadurch gekennzeichnet, dass** die Saugleitung (101) eine vorgefertigte Form und Größe hat, die es erlaubt, sie leicht am Boden des Mundes zu platzieren und gleichzeitig den unteren Teil des Unterkiefers für eine sichere Fixierung zu bedecken, und dass sich der Saugteil (103) entlang des unteren Teils des Unterkiefers erstreckt, um zwischen der Zunge und dem Unterkiefer der Person festgeklemmt zu werden.

2. Dentale Absauganordnung nach Anspruch 1, **dadurch gekennzeichnet, dass** ein transversaler Abschnitt (115) in der Saugleitung (101) im Anschluss an den U-förmigen Saugteil (103) gebildet ist, wobei der transversale Abschnitt (115) geeignet ist, einen Kieferbereich distal der Zahnreihe zu durchqueren.

3. Dentale Absauganordnung nach Anspruch 1, **dadurch gekennzeichnet, dass** die Saugleitung (101) an einem transversalen Abschnitt (115) einen abgeflachten Querschnitt und an anderer Stelle bevorzugt einen im Wesentlichen kreisförmigen Querschnitt aufweist.

4. Dentale Absauganordnung nach einem der vorhergehenden Ansprüche, **dadurch gekennzeichnet, dass** die Saugleitung (101) zur Anpassung an eine Anatomie verformbar ist und bevorzugt dass der ein U bildende Saugteil (103) verformbar ist, insofern als mindestens ein Seitenschenkel des U in die gewünschte Form gebogen werden kann.

5. Dentale Absauganordnung nach einem der vorhergehenden Ansprüche, **dadurch gekennzeichnet, dass** an mindestens einem Abschnitt der Saugleitung (101) oder des Saugteils (103) oder des Absorptionskörpers (111) mindestens ein Zungenhalter und/oder ein Zungenschild (114) oder Schutzschild (114) befestigt ist.

6. Dentale Absauganordnung nach einem der vorhergehenden Ansprüche, **dadurch gekennzeichnet, dass** der Absorptionskörper (111), in einer Querschnittsansicht senkrecht zur Längserstreckung des Absorptionskörpers (111), eine nicht kreisförmige Kontur aufweist und/oder eine Kontur mit einer Basis und einer Oberseite aufweist, wobei die Basis breiter ist als die Oberseite, und/oder eine Kontur mit mindestens einem konkaven Teil aufweist.

7. Dentale Absauganordnung nach einem der vorhergehenden Ansprüche, **dadurch gekennzeichnet, dass** der Absorptionskörper (111) ein Verlängerungsteil umfasst, das ein Teil des Absorptionskörpers (111) oder ein separates Teil aus einem Beflockungsmaterial oder Fasern ist, wobei sich das Verlängerungsteil, bevorzugt wenn es in dem Hohlraum platziert ist, distal von der Saugleitung (101) erstreckt, wobei das Verlängerungsteil bevorzugt eine reduzierte Querschnittsfläche aufweist, die in Richtung seines distalen Endes abnimmt.

8. Dentale Absauganordnung nach einem der vorhergehenden Ansprüche, **dadurch gekennzeichnet, dass** der Absorptionskörper (111) in seiner Länge einstellbar, bevorzugt duktil verformbar, ist und/oder einen Basiskörper mit einem Stanzmuster aufweist, wodurch ermöglicht wird, dass der Körper zusammen mit einem Schaumstoffkörper des Absorptionskörpers verlängert wird.

9. Dentale Absauganordnung nach einem der vorhergehenden Ansprüche, **dadurch gekennzeichnet, dass** sie eine sich vom Hauptabsorptionskörper (111) erstreckende Sekundärsaugleitung (101) aus einstellbarem Material zur Verbindung mit einem Sekundärabsorptionskörper (111) umfasst und/oder dass die Absauganordnung mehrere Absorptionskörper umfasst und dass die Saugleitung zusätzliche Leitungen umfasst, wodurch jede zusätzliche Leitung mit einem Saugteil im jeweiligen Absorptionskörper verbunden ist, bevorzugt zum Ansaugen an der Glandula parotis, an einer Seite davon oder an beiden Seiten davon.

10. Dentale Absauganordnung nach einem der vorhergehenden Ansprüche, **dadurch gekennzeichnet, dass** sie ferner mindestens einen Schutzschild (114) umfasst, um ein drittes Teil daran zu hindern, sich in Richtung eines beliebigen Teils der dentalen Absauganordnung zu bewegen, der bevorzugt in Form und/oder Größe einstellbar ist.

11. Dentale Absauganordnung nach einem der Ansprüche 6 bis 10, **dadurch gekennzeichnet, dass** der Hohlraum eine Mundhöhle ist, ein erhöhter Abschnitt ein Unterkiefer ist, der Schutzschild ein Zungenschutz ist und/oder der Sekundärabsorptionskörper so angeordnet ist, dass er am Ohrspeicheldrüsengang positioniert ist und/oder der Boden der Bereich zwischen der Zunge und dem Boden des Mundes ist.

12. Dentale Absauganordnung nach einem der vorhergehenden Ansprüche, **dadurch gekennzeichnet, dass** ein Schaumstoffkörper entlang der Saugleitung (101) auf dem Gegenbogen (116) platziert ist, insbesondere zur Befestigung außerhalb des Mundes unter dem Kinn, und/oder dass beide Enden der Saugleitung (101, 120) Gegenbögen (116) bilden, insbesondere zur Verlängerung unter dem Kinn zur beidseitigen Befestigung des U-förmigen und/oder parabelförmigen Absorptionskörpers (111) .

13. Dentale Absauganordnung nach einem der vorhergehenden Ansprüche, **dadurch gekennzeichnet, dass** sich ein Metalldraht oder ein anderes Versteifungselement innerhalb der Saugleitung (101) oder mindestens innerhalb des äußeren Umfangs der Saugleitung (101) erstreckt, um eine mögliche Anpassung der Form der Saugleitung (101) zu verbessern und/oder dass ein Feuchtigkeitssensor (118) oder ein Durchflussmesser (118) an der Saugleitung (101) oder dem Absorptionskörper (111) vorgesehen ist, der mit einem Draht (119) versehen ist oder als drahtlose Vorrichtung vorgesehen ist.

## Revendications

1. Dispositif d'aspiration dentaire pour l'élimination de la salive et d'autres fluides, où le dispositif d'aspiration comprend une ligne d'aspiration (101) et un corps d'absorption (111) à placer dans la cavité buccale d'une personne pour l'élimination de la salive et d'autres fluides, où la ligne d'aspiration (101) comprend une partie d'aspiration (103) qui est disposée dans le corps d'absorption (111) et qui est équipée d'éléments de trou (104) communiquant avec le corps d'absorption (111), où le corps d'absorption (111) et la ligne d'aspiration (101) qui le traverse, c'est-à-dire la partie d'aspiration (103), sont essentiellement en forme de U et/ou en forme de parabole, la ligne d'aspiration (101) sort du corps d'absorption (111) à l'une de ses extrémités, **caractérisé en ce que** la ligne d'aspiration (101) a une forme et une taille préfabriquées qui lui permettent d'être placée facilement au fond de la bouche et en même temps de couvrir la partie inférieure de la mâchoire inférieure pour une fixation sûre, et **en ce que** la partie d'aspiration (103) s'étend le long de la partie inférieure de la mâchoire inférieure de manière à être serrée entre la langue et la mâchoire inférieure de la personne.

2. Dispositif d'aspiration dentaire selon la revendication 1, **caractérisé en ce qu'**une partie transversale (115) est formée dans la conduite d'aspiration (101), à la suite de la partie d'aspiration en forme de U (103), ladite partie transversale (115) étant apte à traverser une mâchoire distale de la rangée de dents.

3. Dispositif d'aspiration dentaire selon la revendication 1, **caractérisé en ce que** la conduite d'aspiration (101) présente une section transversale aplatie au niveau d'une partie transversale (115) et de préférence une section transversale essentiellement circulaire ailleurs.

4. Dispositif d'aspiration dentaire selon l'une des revendications précédentes, **caractérisé en ce que** la ligne d'aspiration (101) est déformable pour l'adapter à une anatomie, et de préférence **en ce que** ladite partie d'aspiration (103) formant un U est déformable **en ce qu'**au moins une branche latérale du U peut être pliée dans la forme souhaitée.

5. Dispositif d'aspiration dentaire selon l'une des revendications précédentes, **caractérisé en ce qu'**au moins un porte-langue et/ou un protège-langue (114) ou un bouclier de protection (114) est fixé à au moins une partie de ladite conduite d'aspiration (101) ou de la partie d'aspiration (103) ou du corps d'absorption (111).

6. Dispositif d'aspiration dentaire selon l'une des revendications précédentes, **caractérisé en ce que** le corps d'absorption (111), dans une vue en coupe transversale perpendiculaire à l'étendue longitudinale du corps d'absorption (111), a un contour non circulaire et/ou a un contour avec une base et un sommet, où la base mentionnée est plus large que le sommet mentionné, et/ou a un contour avec au moins une partie concave.

7. Dispositif d'aspiration dentaire selon l'une quelconque des revendications précédentes, **caractérisé en ce que** le corps d'absorption (111) comprend une partie d'extension qui est une partie du corps d'absorption (111) ou une partie séparée fabriquée à partir d'un matériau de flocage ou de fibres, ladite partie d'extension, de préférence lorsqu'elle est placée dans la cavité, s'étend à distance de la conduite d'aspiration (101), où la partie d'extension a de préférence une zone de section transversale réduite qui diminue en direction de son extrémité distale.

8. Dispositif d'aspiration dentaire selon l'une quelconque des revendications précédentes, **caractérisé en ce que** le corps d'absorption (111) est réglable dans sa longueur, de préférence en étant déformé de manière ductile, et/ou a un corps de base avec un motif de poinçonnage permettant à ce corps ainsi qu'à un corps en mousse du corps d'absorption d'être étendus.

9. Dispositif d'aspiration dentaire selon l'une quelconque des revendications précédentes, **caractérisé en ce qu'**il comprend une conduite d'aspiration secondaire (101) en matériau réglable s'étendant à partir du corps d'absorption principal (111) pour la connexion avec un corps d'absorption secondaire (111) et/ou **en ce que** le dispositif d'aspiration comprend plusieurs corps d'absorption et que la conduite d'aspiration comprend des conduites supplémentaires, où chaque conduite supplémentaire est connectée à une partie d'aspiration dans le corps d'absorption respectif, de préférence pour l'aspiration au niveau de la *glandula parotis,* d'un côté de celle-ci ou des deux côtés de celle-ci.

10. Dispositif d'aspiration dentaire selon l'une quelconque des revendications précédentes, **caractérisé en ce qu'**il comprend en outre au moins un bouclier de protection (114) pour empêcher une troisième partie de se déplacer vers une partie quelconque du dispositif d'aspiration dentaire, de préférence réglable en forme et/ou en taille.

11. Dispositif d'aspiration dentaire selon l'une quelconque des revendications 6 à 10, **caractérisé en ce que** ladite cavité est une cavité buccale, une partie surélevée est une mâchoire inférieure, ledit écran de protection est une protection de la langue et/ou ledit corps d'absorption secondaire est conçu pour être positionné au niveau du canal de la glande parotide et/ou le fond étant la zone située entre la langue et le fond de la bouche.

12. Dispositif d'aspiration dentaire selon l'une quelconque des revendications précédentes, **caractérisé en ce qu'**un corps en mousse est placé le long de la conduite d'aspiration (101) sur le contre-arc (116), en particulier pour être fixé à l'extérieur de la bouche sous le menton, et/ou **en ce que** les deux extrémités de la conduite d'aspiration (101,120) forment des contre-arc (116), en particulier pour s'étendre sous le menton pour une fixation double face du corps d'absorption (111) en forme de U et/ou de parabole.

13. Dispositif d'aspiration dentaire selon l'une quelconque des revendications précédentes, **caractérisé en ce qu'**un fil métallique ou un autre élément de renforcement s'étend à l'intérieur de la ligne d'aspiration (101) ou au moins à l'intérieur de la circonférence extérieure de la ligne d'aspiration (101), pour améliorer un ajustement possible de la forme de la ligne d'aspiration (101) et/ou **en ce qu'**un capteur d'humidité (118) ou un débitmètre (118) est prévu sur la ligne d'aspiration (101) ou le corps d'absorption (111) qui est alimenté par un fil (119) ou est prévu comme un dispositif sans fil.
